# EUROPÄISCHE PATENTSCHRIFT

(11) **EP 4 591 815 B1**
(45) Veröffentlichungstag und Bekanntmachung des Hinweises auf die Patenterteilung: **22.04.2026**
(21) Anmeldenummer: 24153476.7
(22) Anmeldetag: 23.01.2024
(51) Int. Cl.: A61B 18/12, A61B 18/04, A61B 18/14

(54) **GENERATOR MIT PLASMALÄNGENANZEIGE**
GENERATOR WITH PLASMA LENGTH DISPLAY
GÉNÉRATEUR AVEC AFFICHAGE DE LONGUEUR DE PLASMA

(43) Veröffentlichungstag der Anmeldung: 30.07.2025
(73) Patentinhaber: Erbe Elektromedizin GmbH, 72072 Tübingen (DE)
(72) Erfinder: DORNHOF, Konstantin, 72160 Horb am Neckar (DE)
(74) Vertreter: Rüger Abel Patentanwälte PartGmbB

(56) Entgegenhaltungen:
- US-A1- 2011 060 329
- US-A1- 2014 018 795
- US-A1- 2015 133 912
- US-A1- 2018 063 937
- US-A1- 2018 368 905
- US-A1- 2022 313 345
- US-A1- 2023 079 047
- US-A1- 2023 079 881

## Beschreibung

Die Erfindung betrifft einen elektrochirurgischen Generator, der zur Durchführung HF-chirurgischer Anwendungen eingerichtet ist.

Elektrochirurgische Generatoren sind aus dem Stand der Technik grundsätzlich bekannt. Zum Beispiel offenbart die US 2018/0243558 A1 einen Generator, der aus einzelnen Impulsgeneratoren besteht, die ausgangsseitig parallelgeschaltet sind. Mit diesem Generator lassen sich gezielt Spannungsimpulse zur elektrochirurgischen Behandlung eines Patienten erzeugen.

Weiter offenbart die US 2011/0060329 A1 einen elektrochirurgischen Generator zur Erzeugung einer Behandlungsgleichspannung. Dazu weist der Generator einen Sperrwandler auf, mittels dessen ein Ausgangskondensator geladen wird, auf dem sich eine Gleichspannung aufbaut. Der Generator kann mehrere solcher Blöcke umfassen, um mehrere Lasten zu bestromen.

Aus der EP 4 147 656 A1 geht ein Generator hervor, der mehrere ausgangsseitig in Reihe geschaltete, nach dem Sperrwandlerprinzip arbeitende Generatormodule umfasst. Jedes Generatormodul enthält einen fremdgesteuerten Schalter und einen Transformator, wobei die Primärwicklung des Transformators mit dem Schalter in Reihe geschaltet ist. Die Sekundärwicklungen der verschiedenen Generatormodule sind miteinander in Reihe geschaltet. Der Schalter ist in einer Steuerschaltung fremdgesteuert, sodass er in der Primärwicklung des Transformators gezielt einen Strom aufbauen und abrupt abschalten kann. Dabei entstehen durch Induktion an dem Transformator in der Sekundärwicklung eine große Spannungsspitze und eine dem Übersetzungsverhältnis entsprechend kleinere Spannungsspitze an der Primärwicklung. Um eine Zerstörung des Schalters durch zu große primäre Spannungsspitzen zu verhindern, ist dem Schalter ein Schutzkondensator parallelgeschaltet.

Weiterer Stand der Technik wird durch die US 2022/313345 A1, US 2023/079881 A1, US 2018/063937 A1, US 2015/0133912 A1**,** die US 4,878,493 und die WO 2011/146498 A2 gebildet.

Wenigstens einige der vorgenannten elektrochirurgischen Generatoren sind dazu geeignet, ein Instrument mit Spannung zu versorgen, welches eine Elektrode zur Behandlung von biologischem Gewebe aufweist. Zur Behandlung kann zwischen dem Gewebe und der Elektrode ein elektrischer Funke bzw. ein Plasma aufrechterhalten werden, dessen Länge Einfluss auf die physiologische Wirkung hat. Die Elektrode kann in einem Gasstrom, insbesondere einem Inertgasstrom, beispielsweise einem Argonstrom angeordnet sein, der an der Elektrode entlangstreichend auf das biologische Gewebe gerichtet ist. Solche Instrumente werden als Argonplasmasonden oder Argonplasmainstrumente bezeichnet.

Ein Beispiel eines solchen Argonplasmainstruments ist beispielsweise aus der EP 1 684 653 B1 bekannt. Dieses Instrument weist einen rohr- oder schlauchartigen Grundkörper auf, der an einem an dem distalen Ende mündenden Kanal zur Gas- und Plasmaführung aufweist. In den Kanal ist eine Elektrode zur Ionisierung des Gasstrahls angeordnet, sodass das Instrument einen Plasmastrahl erzeugt, der in distaler Richtung aus dem Instrument austritt.

Weitere Instrumente sind aus der DE 100 30 111 A1 oder der EP 1 293 170 A1 bekannt.

Bei der Behandlung von biologischem Gewebe kann für den Behandler die Sicht auf den Behandlungsort eingeschränkt sein. Damit ist es für den Behandler manchmal schwierig, die Länge des erzeugten Plasmas abzuschätzen. Die Länge des erzeugten Plasmas kann aber erheblichen Einfluss auf das erzielte Behandlungsergebnis haben.

Daraus leitet sich die der Erfindung zugrundeliegende Aufgabe ab, eine Möglichkeit zu schaffen, um dem Behandler die Länge des erzeugten Plasmas auch dann zu vermitteln, wenn die Sicht auf den Behandlungsort und das erzeugte Plasma eingeschränkt ist.

Diese Aufgabe wird mit dem Generator nach Anspruch 1 gelöst:
Der erfindungsgemäße elektrochirurgische Generator ist zur Speisung HF-chirurgischer Instrumente, insbesondere einen Plasmastrahl erzeugende Instrumente, eingerichtet. Dazu weist der Generator mindestens ein Wandlermodul auf, das eingangsseitig an eine Gleichspannung angeschlossen ist, um von dieser mit elektrischer Leistung versorgt zu werden. Das Wandlermodul weist einen Ausgang auf, der zur Abgabe einer Folge von Hochspannungsimpulsen eingerichtet ist. Die Pulsfolgefrequenz kann dabei eine Mindestfrequenz von 100 kHz übersteigen, sodass die Impulsfolge eine hochfrequente Hochspannung bildet. Insbesondere kann eine nichtsinusförmige Hochspannung erzeugt werden.

Das Wandlermodul weist einen Transformator mit einer Primärwicklung und einer Sekundärwicklung auf. Während die Sekundärwicklung den Ausgang des Wandlermoduls bildet, ist die Primärwicklung mit einem fremdgesteuerten elektronischen Schalter verbunden. Dieser weist eine mit einer Steuerschaltung verbundene Steuerelektrode und eine Steuerstrecke auf, die einen Anschluss der Primärwicklung mit Masse (gesteuert) verbindet. Der andere Anschluss der Primärwicklung ist mit der Betriebsspannung verbunden.

Zwischen der Steuerstrecke und dem an die Steuerstrecke angeschlossenen Ende der Primärwicklung ist ein Verbindungspunkt gebildet, an dem ein Spannungsdetektor angeschlossen ist. Dieser Spannungsdetektor ist dazu eingerichtet, die an diesem Verbindungspunkt auftretende Spannung zu erfassen. Der Spannungsdetektor weist außerdem eine Indikatoreinrichtung auf, die dazu eingerichtet ist, ein von der durch den Spannungsdetektor erfassten Spannung abhängiges Signal zu generieren. Dieses Signal ist ein von dem Behandler wahrnehmbares Signal, beispielsweise ein optisches oder ein akustisches oder auch ein haptisch wahrnehmbares Signal. Auch Kombinationen solcher Signale sind möglich.

Die von dem Spannungsdetektor erfasste Spannung hängt von der Länge des erzeugten Plasmas ab. Dieses Plasma kann sich z.B. zwischen der Elektrode eines an den Generatorausgang angeschlossenen Instruments und biologischem Gewebe ausbilden, das über eine Neutralelektrode ebenfalls an den Generatorausgang angeschlossen ist. Dies gilt für monopolare Instrumente. Es sind jedoch auch bipolare Instrumente mit zwei Elektroden möglich, die an den Generator angeschlossen sind und zwischen denen das Plasma ausgebildet wird.

Der Behandler kann anhand des ihm zugänglich gemachten Signals auf die Länge des an dem Instrument vorhandenen Plasmas Rückschlüsse ziehen und seine Handlungen danach ausrichten. Beispielsweise kann er so die Erzeugung zu großer Plasmalängen oder auch die Erzeugung zu geringer Plasmalängen bis hin zum direkten Kontakt zwischen der Elektrode des Instruments und dem Gewebe vermeiden. Die Erfindung hilft dem Behandler, den passenden Abstand zwischen dem Instrument bzw. der Elektrode und dem Gewebe zu kontrollieren und bei der Behandlung einen zweckmäßigen Abstand einzuhalten.

Der Spannungsdetektor kann ein Spitzenspannungsdetektor sein, der die an dem Verbindungspunkt auftretende Maximalspannung anzeigt. Dieser kann im Weiteren als Sample -and-Hold Schaltung ausgebildet sein, indem die von dem Spannungsdetektor ausgeführte Spannungsmessung mit dem Schalten des fremdgesteuerten Schalters synchronisiert wird. Dazu kann die den Schalter steuernde Steuerschaltung mit dem Spannungsdetektor verbunden sein. So kann der Spannungsdetektor von der Steuerschaltung Steuerimpulse erhalten.

Ist der Spannungsdetektor ein Spitzenspannungsdetektor, enthält er typischerweise einen Speicherkondensator, der über einen Strompfad mit dem Verbindungspunkt verbunden ist, um sich auf die dort anliegende Spitzenspannung aufzuladen. Ist der Spannungsdetektor eine Sample-and-Hold Schaltung, kann dem Speicherkondensator ein Entladestrompfad parallelgeschaltet sein. In diesem kann ein gesteuerter Entladeschalter vorgesehen sein, der von der Steuerschaltung gesteuert wird, um den Speicherkondensator immer kurz vor demjenigen Zeitpunkt zu entladen, an dem der gesteuerte Schalter des Wandlermoduls gesperrt und somit eine neue Spitzenspannung erreicht wird.

Der Generator kann mehrere Wandlermodule der beschriebenen Bauart aufweisen. Vorzugsweise sind diese Wandlermodule parallel zueinander von einer Gleichspannung mit Energie bzw. Leistung versorgt. Ausgangsseitig sind die Wandlermodule vorzugsweise in Reihe geschaltet. Jedes Wandlermodul kann einzeln "feuern", d.h. an seinem Ausgang einen Hochspannungsimpuls abgeben, wenn sein gesteuerter Schalter kurzzeitig sperrt. Eine "Feuersequenz" ist dann eine Folge von Hochspannungsimpulsen, bei denen jedes an der Feuersequenz beteiligte Wandlermodul ein oder mehrmals gefeuert hat. Der Feuersequenz entspricht eine Sequenz von Steuersignalen, die die Steuerschaltung erzeugt und an die Schalter der Wandlermodule leitet. Die Steuerschaltung kann darauf eingerichtet sein, zur Erzeugung einer HF-Ausgangsspannung die Sequenz der Steuersignale periodisch beliebig oft zu wiederholen. Entsprechend entsteht die HF-Ausgangsspannung durch fortwährende Wiederholung der Feuersequenz.

Die Steuerschaltung ist vorzugsweise darauf eingerichtet, die gesteuerten Schalter der Wandlermodule während einer Feuersequenz einzeln oder in Gruppen zu sperren, wobei die anderen Schalter der anderen Wandlermodule dann vorzugsweise durchlässig gehalten werden. Dadurch sind die Hochspannungsausgänge der gerade nicht feuernden Wandlermodule durchlässig für die Hochspannungsimpulse der feuernden Wandlermodule. Ein Wandlermodul feuert, wenn sein gesteuerter Schalter (vorzugsweise kurzzeitig) gesperrt wird und auf diese Weise ein Hochspannungsausgangsimpuls entsteht.

Jedes dieser Wandlermodule kann einen Spannungsdetektor nach der beschriebenen Bauart aufweisen. Es hat sich aber gezeigt, dass es genügen kann, ein einziges Wandlermodul oder nur einige der Wandlermodule mit dem genannten Spannungsdetektor zu verbinden.

Weitere Einzelheiten von Ausführungsformen der Erfindung gehen aus Unteransprüchen sowie der Beschreibung und der zugehörigen Zeichnung hervor. In dieser zeigen:
Figur 1 den erfindungsgemäßen Generator und ein an diesen angeschlossenes Instrument, in Prinzipdarstellung,
Figur 1a das distale Ende eines Instruments während des Betriebs in längs geschnittener Darstellung,
Figur 2 einen Generator mit einem einzigen Wandlermodul und Plasmalängenanzeige in einem schematisierten Schaltbild,
Figur 3 einen Generator mit mehreren Wandlermodulen und einem Spannungsdetektor, in Prinzipdarstellung,
Figur 4 eine abgewandelten Generator gemäß Figur 3, in Prinzipdarstellung,
Figur 5 bis 8 Schaltdiagramme und Spannungen des Generators nach Figur 2 bis 4,
Figur 9 einen Zusammenhang zwischen der von dem Spannungsdetektor erfassten Spannung und der Plasmalänge,
Figur 10 Beispiele für Feuersequenzen der Wandlermodule und den Betrieb des Spannungsdetektors.

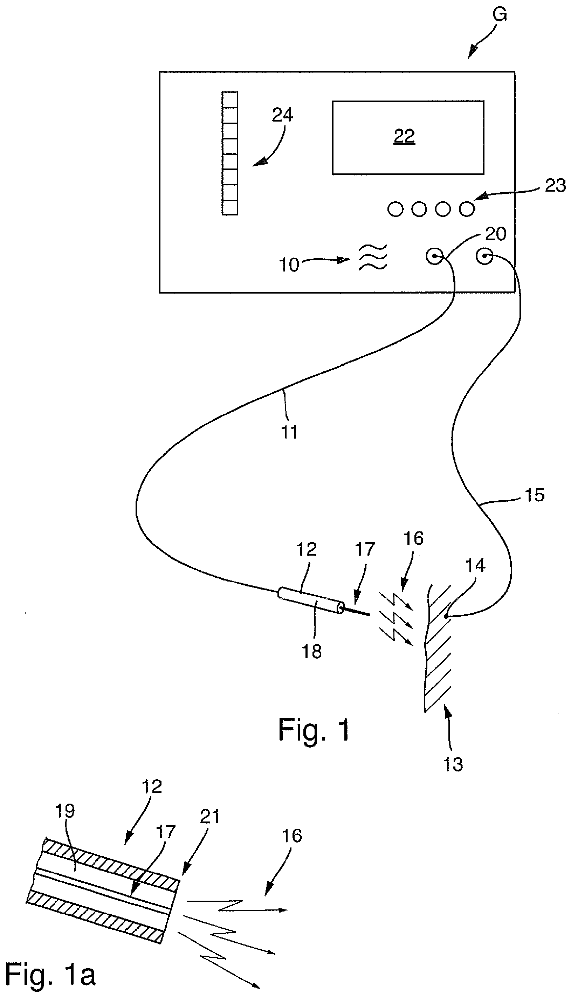

In Figur 1 ist ein Gerät G mit einem erfindungsgemäßen Generator 10 veranschaulicht, an den über eine Leitung 11 ein Instrument 12 zur Behandlung von biologischem Gewebe 13 angeschlossen ist. Das biologische Gewebe 13 ist über eine Neutralelektrode 14 und eine Leitung 15 mit dem Generator 10 verbunden. Figur 1 veranschaulicht somit ein monopolares Instrument, bei dem Strom von dem Instrument 12 zu dem Gewebe 13 fließt. Prinzipiell ist die Erfindung jedoch auch auf bi- oder mehrpolare Instrumente anwendbar, die mittels eines Plasmas 16 auf biologisches Gewebe einwirken. In Figur 1 ist das Plasma 16 durch gezackte Pfeile veranschaulicht.

Das Instrument 12 weist zur Plasmaerzeugung mindestens eine Elektrode 17 auf, die elektrisch über die Leitung 11 mit dem Generator 10 verbunden ist. In Figur 1 ist das Instrument 12 als Handgriff mit davon abstehender Elektrode 17 veranschaulicht. Solche Instrumente eignen sich für den offenchirurgischen Einsatz. Die Elektrode 17 kann dabei von dem Handgriff 18 weg ragen und nach Art einer Nadel oder eines Skalpells ausgebildet sein. Es ist jedoch auch möglich, die Elektrode 17 in einem Kanal 19 anzuordnen, der sich durch das Instrument 12 erstreckt und an dem proximalen Ende 20 an das speisende Gerät G angeschlossen ist, das dann außer dem Generator 10 eine entsprechende Gasversorgung aufweist.

An dem distalen Ende 21 (Figur 1a) kann das von dem Gerät G in den Kanal 19 eingeleitete Gas ausströmen. Es kann über die Elektrode 17 ionisiert sein, sodass sich ein ausströmender Plasmastrom 16 bildet, der wiederum durch gezackte Pfeile veranschaulicht ist. Dabei kann das Instrument 12, wie in Figur 1 dargestellt, sowohl als für den offenchirurgischen Gebrauch vorgesehenes Instrument, als auch als laparoskopisches Instrument, wie auch als Sonde ausgebildet sein, die beispielsweise durch einen Arbeitskanal eines Endoskops an den Operationsort geführt wird. All diesen Anwendungen ist aber gemeinsam, dass der Behandler nicht immer uneingeschränkt freie Sicht auf den Plasmastrom 16 und dessen Länge hat.

Das Gerät G weist, wie Figur 1 veranschaulicht, sowohl eine Anzeigeeinrichtung 22 als auch Bedienelemente 23 auf, beispielsweise Tasten, Knöpfe und dergleichen. Zusätzlich weist das Gerät G eine Indikatoreinrichtung 24 auf, die zur Anzeige der Länge des Plasmastroms 16 dient. Die Indikatoreinrichtung 24 ist in Figur 1 als optische Indikatoreinrichtung dargestellt, die einen Leuchtbalken anzeigt, der die Länge des Plasmastroms 16 symbolisiert. Außerdem kann der Leuchtbalken an mindestens einem seiner Enden farbige Felder aufweisen, mit denen er einen zu langen und/oder zu kurzen Plasmastrom 16 signalisiert.

Die Indikatoreinrichtung 24 kann als optische Indikatoreinrichtung ausgebildet und bedarfsweise auch in die Anzeigeeinrichtung 22 integriert sein. Weiter ist es möglich, die Indikatoreinrichtung 24 zusätzlich oder alternativ mit akustischen Anzeigemitteln zu versehen. Zusätzlich oder alternativ kann die Indikatoreinrichtung 24 dazu ausgebildet sein, haptisch wahrnehmbare Signale zu generieren, so z.B. in dem Griff 18 oder andernorts spürbare Vibrationen.

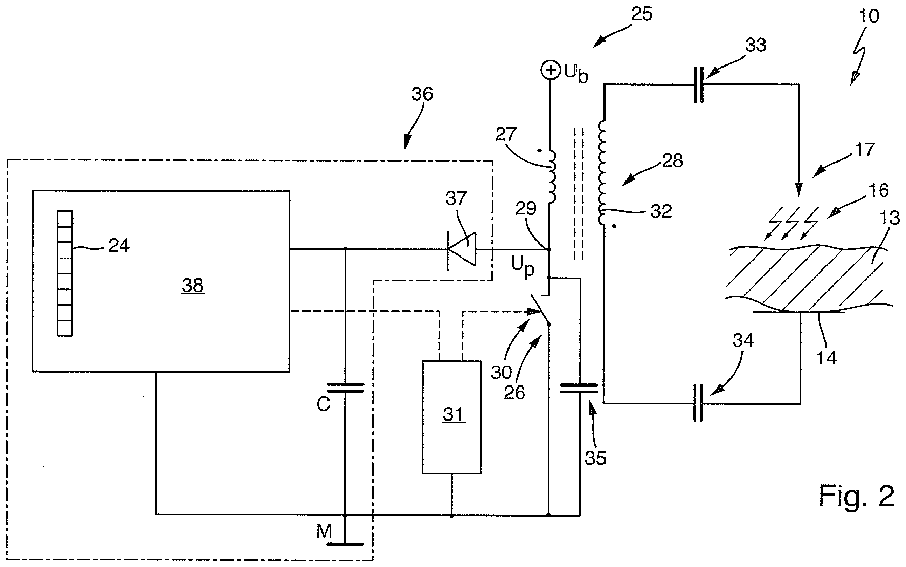

Figur 2 veranschaulicht einen Teil der elektrischen Schaltung des Generators 10 in vereinfachter Darstellung. Die bereits eingeführten Bezugszeichen werden nachfolgend mit gleicher Bedeutung weiterverwendet.

Der elektrochirurgische Generator 10 umfasst ein Wandlermodul 25, zu welchem eine nach Art eines Sperrwandlers ausgebildete elektrische Schaltung gehört. Diese wird durch einen fremdgesteuerten elektronischen Schalter 26 beispielsweise in Gestalt des Feldeffekttransistors oder eines Bipolartransistors oder eines Bipolartransistors mit isoliertem Gate oder einen sonstigen elektronischen Schalter gebildet, der mit einer Primärwicklung 27 eines Transformators 28 in Reihe verbunden ist. Dabei ist ein erstes Ende der Primärwicklung 27 mit einer positiven Betriebsspannung U_{b} verbunden, während das andere Ende der Primärwicklung 27 mit einem Verbindungspunkt 29 verbunden ist. An diesen Verbindungspunkt 29 ist der fremdgesteuerte elektronische Schalter 26 mit einem Ende (Drain oder Collektor) verbunden, während das andere Ende seiner Steuerstrecke (Source oder Emitter) mit Massepotenzial M verbunden ist.

Der fremdgesteuerte elektronische Schalter 26 weist außerdem eine Steuerelektrode 30 auf, die mit einer Steuerschaltung 31 verbunden ist, um die Steuerstrecke des elektronischen Schalters 26 kontrolliert freizugeben oder zu sperren.

Der Transformator 28 weist außerdem eine Sekundärwicklung 32 auf, deren eines Ende gegebenenfalls über einen Koppelkondensator 33 mit der Elektrode 17 des Instruments 12 verbunden ist. Das andere Ende der Sekundärwicklung ist gegebenenfalls über einen weiteren Koppelkondensator 34 mit der Neutralelektrode 14 verbunden, die am Patienten und somit am biologischen Gewebe 13 zu befestigen ist. Die Sekundärwicklung 32 oder, falls vorhanden, die von der Sekundärwicklung abgewandten Elektroden der Koppelkondensatoren 33, 34 bilden den Ausgang des Wandlermoduls 25 und somit des Generators 10.

Dem elektronischen Schalter 26 kann ein Schutzkondensator 35 oder auch eine anderweitige Schutzbeschaltung parallelgeschaltet sein. Der Schutzkondensator 35 ist mit einem Anschluss an den Verbindungspunkt 29 und mit seinem anderen Anschluss an Masse angeschlossen. Er dient dazu, die Spannung an dem Schalter 26 zu begrenzen.

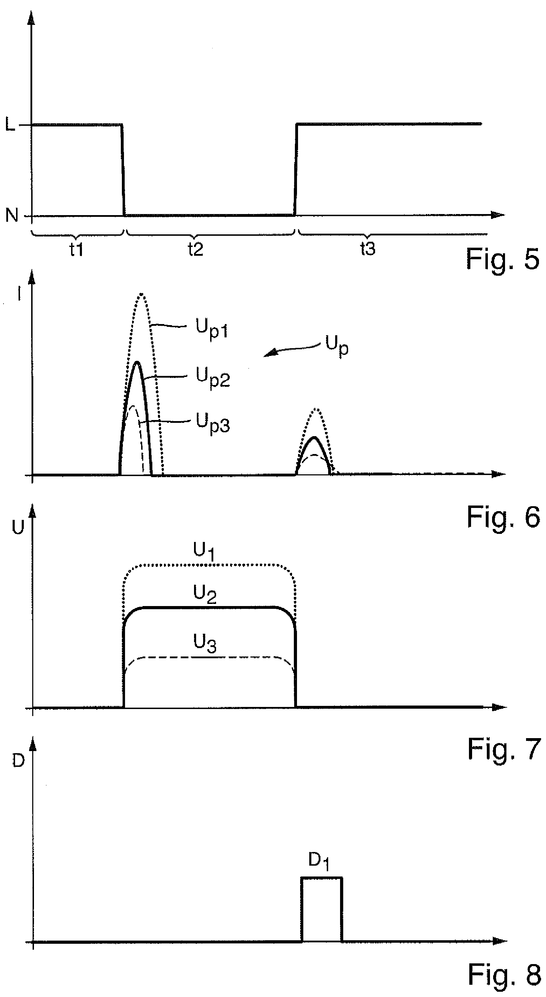

An den Verbindungspunkt 29 ist ein Spannungsdetektor 36 angeschlossen, der dazu eingerichtet ist, die an dem Verbindungspunkt 29 und somit an dem Schalter 26 (wie auch dem Schutzkondensator 35) auftretende Spannung U_{P} zu erfassen. Zu dem Spannungsdetektor 36 gehört die Indikatoreinrichtung 24, die dazu dient, ein Signal zu erzeugen, das von der erfassten Spannung U_{P} (Figur 6) abhängig ist und deren Größe kennzeichnet. Die Indikatoreinrichtung 24 kann beispielsweise an dem Gerät G bzw. Generator 10 oder auch an dem Instrument 12 oder an einem gesonderten Ort angeordnet sein.

Vorzugsweise ist der Spannungsdetektor 36 als integrierender Detektor oder als Spitzenwertdetektor ausgebildet. Er weist einen Speicherkondensator C auf, der über eine Ladeschaltung 37 mit dem Verbindungspunkt 29 verbunden ist. Im einfachsten Fall ist die Lageschaltung 37 eine bezüglich der an dem Verbindungspunkt 29 auftretenden Spannung Uₚ in Flussrichtung gepolte Diode. Vorzugsweise wird eine besonders kapazitätsarme Diode verwendet. Zur Verringerung der parasitären Kapazität der Ladeschaltung 37 können mehrere Dioden miteinander in Reihe geschaltet werden.

Dem Speicherkondensator C ist eine Auswerteschaltung 38 parallelgeschaltet, die einerseits die Spannung des Kondensators C mittels der Indikatoreinrichtung 24 anzeigt und andererseits dazu eingerichtet ist, den Speicherkondensator C periodisch zu entladen.

Der insoweit beschriebene Generator arbeitet wie folgt:
Zur Behandlung des Gewebes 13 wird die Elektrode 17 in die Nähe des Gewebes 13 gebracht. Die Steuerschaltung 31 schließt und öffnet den Schalter 26 nun in schneller Folge und lässt das Wandlermodul 25 auf diese Weise feuern. Ist der Schalter 26 durchlässig, fließt ein Strom von der (z.B. positiven) Betriebsspannung U_{b} durch die Primärwicklung 27 von dem durch einen Punkt gekennzeichneten Spulenanfang zu dem Spulenende und somit über den Verbindungspunkt 29 und den leitenden Schalter 26 nach dem Massepotenzial M ab. Immer wenn die Steuerschaltung 31 einen Sperrimpuls an die Steuerelektrode 30 gibt, sperrt der Schalter 36, sodass ein weiterer Stromfluss nach Masse unmöglich wird. Der Stromfluss kommutiert nun auf die Sekundärwicklung 32, wo der Strom weiterhin von dem durch den Punkt gekennzeichneten Spulenanfang zu dem Spulenende (und somit zu dem Koppelkondensator 33) und über diesen zu der Elektrode 17 fließt.

Durch Funkenüberschlag zu dem Gewebe 13 infolge der erzeugten Hochspannungsimpulse entsteht das Plasma 16, das entweder in Luft, Dampf oder auch einem eigens herbeigeführten Gas, beispielsweise Argon, brennen kann. Die zwischen der Elektrode 17 und dem Gewebe 13 bzw. der Neutralelektrode 14 sich aufbauende Spannung ist dabei ein Maß für die Länge des Plasmas 16. Die zwischen der Elektrode 17 und der Neutralelektrode 14 anstehende Spannung steht auch an der Sekundärwicklung 32 an. Durch entsprechend dem Übersetzungsfaktor des Transformators 28 steht die entsprechende Spannung Uₚ auch an der Primärwicklung 27 und als entsprechender Spannungsimpuls somit an dem Verbindungspunkt 27 an. Zur Erläuterung wird auf die Diagramme nach Figur 5 und 6 verwiesen:
In Figur 5 ist der Schalter 26 während des Zeitabschnitts t1 leitend. Der leitende Zustand ist auf der Ordinate mit dem Großbuchstabe L gekennzeichnet. Am Ende der Zeitspanne t1 wird der Schalter 26 gesperrt. Es beginnt die Sperrphase, die in Figur 5 mit der Zeitspanne t2 gekennzeichnet ist. Der nichtleitende Zustand ist an der Ordinate durch den Buchstaben N charakterisiert. Zu Beginn der Sperrphase t2 entsteht der beschriebene Spannungsimpuls Uₚ an der Primärwicklung 27 und entsprechend überhöht auch an der Sekundärwicklung 32. Dieser Spannungsimpuls Uₚ ist in Figur 6 in drei verschiedenen Größen Uₚ₁, Uₚ₂, Uₚ₃ veranschaulicht, was drei verschiedenen Plasmalängen des Plasmas 16 entspricht. Dabei zeigt sich, dass der Spannungsimpuls Uₚ umso größer ist, je länger das Plasma 16 ist.

Figur 7 veranschaulicht dazu die Spannung U auf dem Speicherkondensator C. Über die Ladeschaltung 37 fließt während des Spannungsimpulses Uₚ Strom auf den Speicherkondensator C und lädt diesen auf eine entsprechende Spannung U1, U2 oder U3 auf. Je größer der Spannungsimpuls Uₚ ist, desto größer ist die auf dem Speicherkondensator C anstehende Spannung U.

Der Speicherkondensator C kann bedarfsweise von Zeit zu Zeit entladen werden. Beispielsweise kann er vor, während oder nach dem Wiedereinschalten des Schalters 26, das gemäß Figur 5 in der Leitphase t3 stattfindet, entladen werden. Figur 8 veranschaulicht einen solchen Entladeimpuls D1.

Die Indikatoreinrichtung 24 ist darauf eingerichtet, ein der Spannung U1, U2 oder U3 entsprechendes Signal zu erzeugen, beispielsweise indem ein von ihr wiedergegebener Leuchtbalken seine Länge entsprechend der Spannung U auf dem Speicherkondensator C in Stufen oder kontinuierlich verändert.

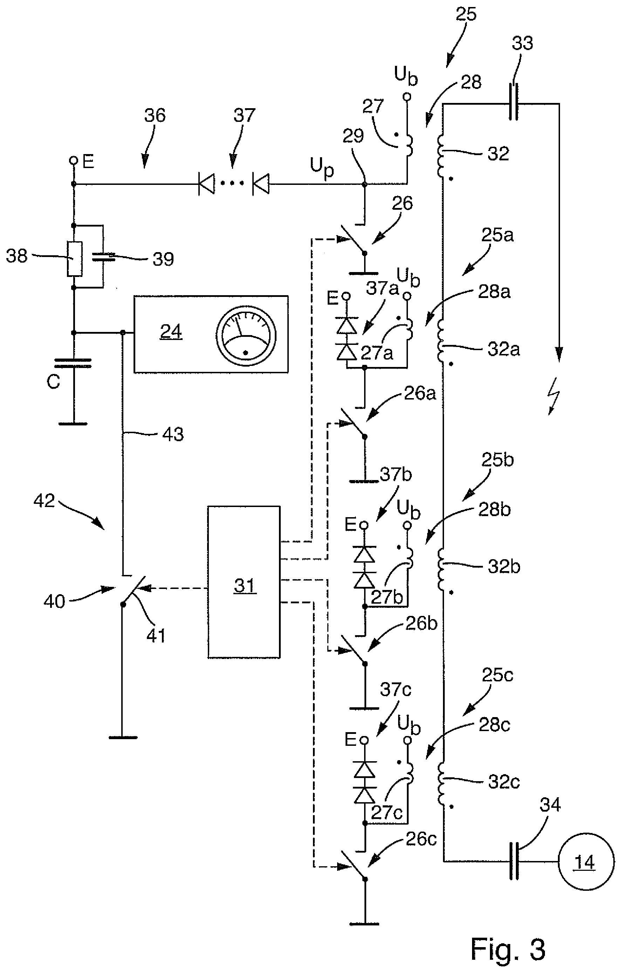

Figur 3 veranschaulicht eine weiterentwickelte Form des Generators nach Figur 2, wobei die vorstehende Beschreibung unter Beibehaltung der bereits eingeführten Bezugszeichen und zusätzlicher Berücksichtigung der nachfolgenden Erläuterung gilt:
Der Generator nach Figur 3 weist mehrere Wandlermodule 25, 25a, 25b, 25c auf, wobei die Wandlermodule 25a, 25b, 25c vorzugsweise mit dem Wandlermodul 25 baugleich sind und die vorige Beschreibung unter Hinzunahme eines jeweiligen Buchstabenindizes entsprechend gilt.

Die Ladeschaltung 37 ist mindestens an den Verbindungspunkt 29 angeschlossen und führt zunächst über eine Kettenschaltung von in Flussrichtung gepolten Dioden zu einem Schaltungspunkt E. Von diesem Schaltungspunkt E aus führt der Strompfad vorzugsweise über einen Widerstand 38 und einen parallel geschalteten Kondensator 39 zu dem Speicherkondensator C. Die Indikatoreinrichtung 24 ist an diesen Speicherkondensator C angeschlossen.

Zur gelegentlichen, regelmäßigen oder bedarfsweisen Entladung des Speicherkondensators C ist eine Entladeschaltung 42 mit einem Entladestrompfad 43 vorgesehen, in dem ein Entladeschalter 40 angeordnet ist. Der Entladeschalter 40 weist eine Steuerstrecke auf, die dem Speicherkondensator C parallelgeschaltet ist. Seine Steuerelektrode 41 ist mit der Steuerschaltung 31 verbunden, die nun nicht nur alle Schalter 26, 26a, 26b, 26c, sondern auch den Entladeschalter 40 steuert. In diesem Fall symbolisiert der in Figur 8 veranschaulichte Impuls D1, die Zeitspanne während derer der Entladeschalter 40 freigegeben ist, d.h. leitet. Damit lässt er die Ladung von dem Speicherkondensator C abfließen, womit die Spannung U1, U2 oder U3 zusammenbricht, d.h. auf nahe Null fällt.

In vielen Fällen genügt es, wenn die Ladeschaltung 37 lediglich den Verbindungspunkt 29 mit dem Schaltungspunkt E verbindet. Es kann aber sinnvoll sein, auch einen oder weitere der Wandlermodule 25a, 25b, 25c mit entsprechenden Ladeschaltungen 27a, 27b, 27c mit dem Spannungspunkt E zu verbinden.

Die Steuerschaltung 31 kann dazu eingerichtet sein, die Wandlermodule 26, 26a, 26b, 26c aufeinander abgestimmt zu aktivieren und zu deaktivieren, d.h. deren jeweiligen Schalter 26, 26a, 26b, 26c zu öffnen und zu schließen. Dazu können die einzelnen Schalter 26, 26a, 26b, 26c einzeln aus ihrem leitenden Zustand kurzzeitig in die Sperrphase T2 gemäß Figur 5 überführt werden, um an ihrer jeweiligen Sekundärwicklung 32, 32a, 32b, oder 32c zu erzeugen, d.h. zu feuern. Vorzugsweise sind die Schalter derjenigen Wandlermodule 25, 25a, 25b, 25c, die nicht feuern, leitend. Damit kann der von dem jeweils feuernden Wandlermodul 25, 25a, 25b, 25c abgegebene Hochspannungsimpuls durch die dann niederohmigen Sekundärwicklungen der nicht feuernden Wandlermodule hindurchdringen, sodass der Hochspannungsimpuls durch die miteinander in Reihe geschalteten Sekundärwicklungen 32, 32a, 32b, 32c durchfließen kann.

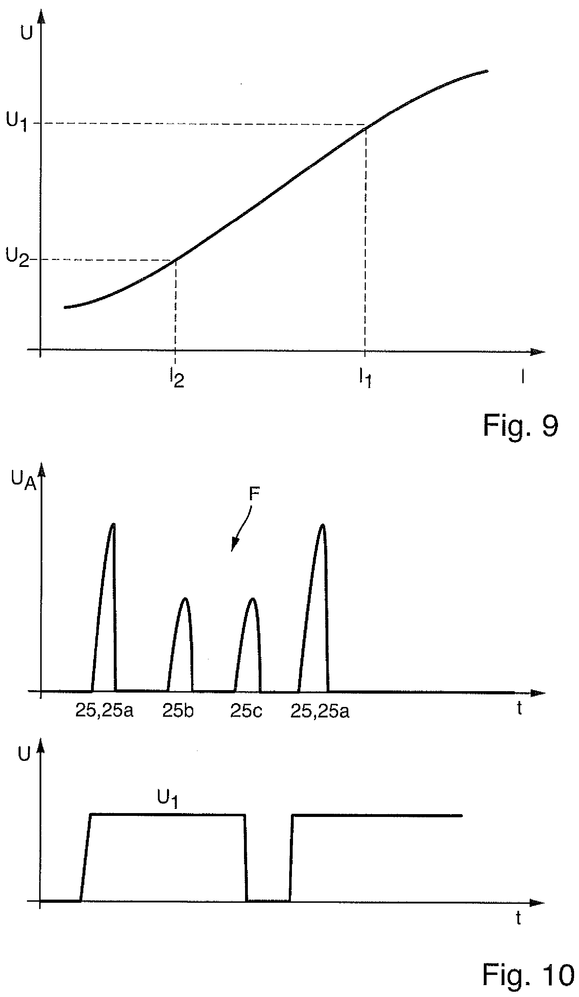

Figur 10 veranschaulicht dazu eine Feuersequenz F anhand der Spannung U₁₇ an der Elektrode 17. Es feuern gleichzeitig die Wandlermodule 25 und 25a, danach die Wandlermodule 25b, 25c. Der an das Wandlermodul 25 angeschlossene Spannungsdetektor 36 erfasst die Spannung Uₚ wobei der Kondensator C entsprechend aufgeladen wird. Die Ladung bleibt bestehen, bis sie wieder gelöscht wird, indem die Steuerschaltung 31 den Entladeschalter 40 aktiviert. Sie kann dies am Ende der Feuersequenz F tun, wenn nur die Spannung Uₚ an einem der Wandlermodule überwacht wird. Sind mehrere oder alle der Wandlermodule 25, 25a, 25b, 25c an den Spannungsdetektor 36 angeschlossen, kann der Kondensator auch jeweils vor dem Feuern des nächsten Wandlermoduls entladen werden.

Typischerweise veranlasst die Steuerschaltung 31 die Wandlermodule 25, 25a, 25b, 25c nach einem vorgegebenen Schema einzeln oder in Gruppen zu feuern. Dabei können Hochspannungsimpulse gleicher oder unterschiedlicher Größe entstehen, wie Figur 10 zeigt. Findet die Entladung des Speicherkondensators C immer erst nach Ablauf einer Feuersequenz statt, wird auf dem Speicherkondensator C eine Spannung aufgebaut, die durch den größten der erzeugten in der Feuersequenz F vorhandenen Hochspannungsimpulse bestimmt ist. Diese Spannung kennzeichnet die Lichtbogenlänge oder Plasmalänge, die mit dem Indikator 24 angezeigt wird.

Figur 9 veranschaulicht den Zusammenhang zwischen der auf dem Speicherkondensator C vorhandenen Spannung U_{C} und der Lichtbogenlänge l. Bei unterschiedlichen Bogenlängen l₁, l₂ ergeben sich unterschiedliche Spannungen U1, U2, wobei der Zusammenhang, zumindest in einem eingeschränkten, jedoch relativ großen Bereich, weitgehend linear ist. Unabhängig von der Linearität ist die Spannung U_{C} ein Maß für die Lichtbogenlänge l.

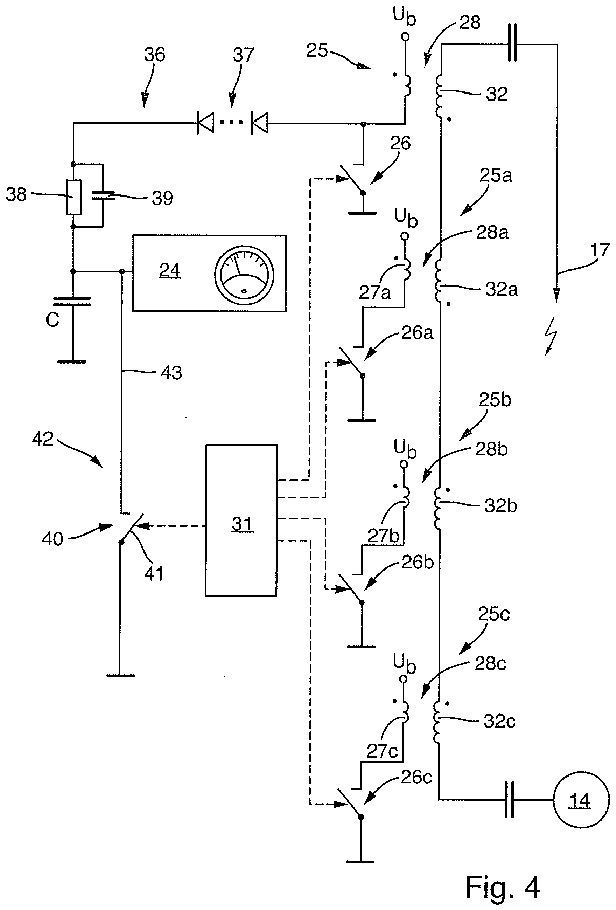

Eine weiter abgewandelte Ausführungsform des erfindungsgemäßen Generators ist in Figur 4 veranschaulicht. Die bereits eingeführten Bezugszeichen gelten auch für diese Ausführungsform, weil die darauf bezogene Beschreibung entsprechend gilt. Der Unterschied zwischen der Ausführungsform nach Figur 4 und der Ausführungsform nach Figur 3 liegt (allein) in der Polung der Sekundärwicklungen 32, 32a, 32b, 32c. Während bei der Ausführungsform nach Figur 3 alle Sekundärwicklungen 32, 32a, 32b, 32b, 32c gleich gepolt sind, indem die durch einen Punkt gekennzeichneten Wicklungsanfänge in Richtung auf die Neutralelektrode 14 hin orientiert sind, sind bei der Ausführungsform nach Figur 4 unterschiedliche Polungen vorhanden. Die Sekundärwicklungen 32, 32a sind mit ihren Wicklungsanfängen der Neutralelektrode 14 zugewandt, während die Sekundärwicklungen 32b, 32c mit ihren Wicklungsanfängen der Elektrode 17 zugewandt sind. Es wird aber darauf hingewiesen, dass auch andere Gruppierungen und Polungen vorgenommen werden können.

Bei der Ausführungsform nach Figur 4 erzeugen die Wandlermodule 25, 25a an der Elektrode 17, wenn sie feuern, positive Spannungsimpulse, während die Wandlermodule 25b, 25c an der Elektrode 17 negative Spannungsimpulse erzeugen. (Dies gilt bei positiver Betriebsspannung U_{b}, bei negativer Betriebsspannung U_{b} sind die Verhältnisse umgekehrt.)

Durch die Zusammenschaltung von Wandlermodulen 25, 25a, 25b, 25c mit unterschiedlicher Polung lassen sich sowohl symmetrische, als auch asymmetrische HF-Ausgangsspannungsfolgen erzeugen. Auch bei symmetrischen HF-Impulsfolgen, die sowohl positiv, als auch negative Spannungsspitzen enthalten, gelingt die Anzeige der Plasmalänge, wie vorstehend beschrieben, durch Erfassung der Spannung Uₚ an den geeigneten Verbindungspunkten 29 und/oder 29a, 29b, 29c.

Der erfindungsgemäße Generator 10 enthält mindestens ein Wandlermodul mit einem Transformator 28, dessen Sekundärwicklung 32 mit der Elektrode 17 eines Instruments 12 und mit einer Gegenelektrode 14 elektrisch verbunden ist. Die Primärwicklung 27 des Transformators 28 liegt einerseits an Betriebsspannung U_{b} und ist andererseits über einen elektronischen Schalter 26 mit Masse M verbunden. Der Schalter 26 sperrt periodisch, wodurch in der Sekundärwicklung 32 Spannungsimpulse erzeugt werden, die einen Funken, oder ein sonstiges Plasma zur medizinischen Behandlung eines Patienten speisen. Zur Anzeige der Plasmalänge dient ein Spannungsdetektor 36, der die an der Primärwicklung 27 auftretende Spannung Uₚ erfasst und über eine Indikatoreinrichtung 24 zur Anzeige bringt. Der Erfindung liegt der Gedanke zugrunde, dass die an der Primärwicklung 27 auftretende Spannung Uₚ, insbesondere die Spitzenspannung, die Länge des erzeugten Plasmas 16 kennzeichnet. Der Erfinder hat herausgefunden, dass andere Einflussfaktoren als die Plasmalänge auf die an der Primärspule 27 gemessene Spannung Uₚ von untergeordneter Bedeutung sind.

### Bezugszeichen:

- 10: Generator
- G: Gerät
- 11: Leitung
- 12: Instrument
- 13: biologisches Gewebe
- 14: Neutralelektrode
- 15: Leitung
- 16: Plasma, Plasmastrom
- 17: Elektrode
- 18: Handgriff
- 19: Kanal
- 20: proximales Ende
- 21: distales Ende
- 22: Anzeigeeinrichtung
- 23: Bedienelemente
- 24: Indikatoreinrichtung
- 25: Wandlermodul
- 26: fremdgesteuerter elektronischer Schalter
- 27: Primärwicklung des Transformators 28
- 28: Transformator
- U_{b}: Betriebsspannung
- 29: Verbindungspunkt
- M: Massepotential
- F: Feuersequenz
- 30: Steuerelektrode
- 31: Steuerschaltung
- 32: Sekundärwicklung
- 33, 34: Koppelkondensatoren
- 35: Schutzkondensator
- 36: Spannungsdetektor
- C: Speicherkondensator
- U_{C}: Spannung auf dem Speicherkondensator C
- 37: Ladeschaltung
- t1, t3: Leitphasen des Schalters 26
- t2: Sperrphase des Schalters 26
- Uₚ, Uₚ₁ - Uₚ₃: Spannungsimpulse
- U, U1 - U3: Spannung auf dem Speicherkondensator
- l, l₁, l₂: Lichtbogenlänge
- E: Schaltungspunkt
- 38: Widerstand
- 39: Kondensator
- 40: Entladeschalter
- 41: Steuerelektrode
- D, D1: Entladeimpuls
- 42: Entladeschaltung
- 43: Entladestrompfad

## Patentansprüche

1. Elektrochirurgischer Generator (10), insbesondere für HF-chirurgische Anwendungen,
mit einem Wandlermodul (25), das einen mit einer Steuerschaltung (31) verbundenen, fremdgesteuerten Schalter (26) und einen Transformator (28) aufweist, dessen Primärwicklung (27) mit dem Schalter (26) in Reihe geschaltet und dessen Sekundärwicklung (32) mit einer Elektrode (17) und einer Gegenelektrode (14) verbunden ist, wobei zwischen der Elektrode (17) und der Gegenelektrode (14) ein zu behandelnder Patient (13) platzierbar ist,
mit einem Spannungsdetektor (36), der mit dem Schalter (26) verbunden und dazu eingerichtet ist, die auftretende Spannung (Uₚ) zu erfassen, und der eine Indikatoreinrichtung (24) aufweist, die dazu eingerichtet ist, ein von der erfassten Spannung (Uₚ) abhängiges Signal zu generieren.

2. Generator nach Anspruch 1, **dadurch gekennzeichnet, dass** der fremdgesteuerte Schalter (26) mit einem Ende mit einem Bezugspotential (M) und mit einem anderen Ende an einen Verbindungspunkt (29) angeschlossen ist, der mit einem Ende der Primärwicklung (27) verbunden ist, wobei der Spannungsdetektor (36) an den Verbindungspunkt (29) angeschlossen ist.

3. Generator nach einem der vorstehenden Ansprüche, **dadurch gekennzeichnet, dass** der Spannungsdetektor (36) ein Spitzenspannungsdetektor ist.

4. Generator nach einem der vorstehenden Ansprüche, **dadurch gekennzeichnet, dass** der Spannungsdetektor (36) eine Sample-and-Hold-Schaltung ist, bei der die von dem Spannungsdetektor (36) ausgeführte Spannungsmessung mit dem Schalten des fremdgesteuerten Schalters (26) synchronisiert wird.

5. Generator nach einem der vorstehenden Ansprüche, **dadurch gekennzeichnet, dass** der Spannungsdetektor (36) einen Speicherkondensator (C) enthält, der über eine Ladeschaltung (37) mit dem Schalter (26) verbunden ist.

6. Generator nach Anspruch 5, **dadurch gekennzeichnet, dass** die Ladeschaltung (37) durch mindestens eine, vorzugsweise mehrere miteinander in Reihe geschaltete Dioden gebildet ist.

7. Generator nach Anspruch 5 oder 6, **dadurch gekennzeichnet, dass** dem Speicherkondensator (C) eine Entladeschaltung (42) zugeordnet ist, die einen steuerbaren Entladestrompfad (43) aufweist, der zu dem Speicherkondensator (C) parallel geschaltet ist.

8. Generator nach Anspruch 7, **dadurch gekennzeichnet, dass** die Entladeschaltung (42) mit der Steuerschaltung (31) verbunden ist, so dass deren Entladestrompfad (43) von der Steuerschaltung (31) zwischen nichtleitendem und leitendem Zustand alternierend hin und her schaltbar ist.

9. Generator nach Anspruch 8, **dadurch gekennzeichnet, dass** die Steuerschaltung (31) darauf eingerichtet ist, den fremdgesteuerten Schalter (26) alternierend zu öffnen und zu sperren, wobei sie weiter dazu eingerichtet ist, auch den Entladestrompfad (43) zu sperren, wenn der Schalter (26) gesperrt ist.

10. Generator nach Anspruch 9, **dadurch gekennzeichnet, dass** die Steuerschaltung (31) darauf eingerichtet ist, den Entladestrompfad (42) vor dem Sperren des Schalters (26) vorübergehend zu öffnen.

11. Generator nach einem der vorstehenden Ansprüche, **dadurch gekennzeichnet, dass** zusätzlich zu dem Wandlermodul (25) mindestens ein weiteres Wandlermodul (25a) vorgesehen ist, wobei die Sekundärwicklungen (32, 32a) der Wandlermodule (25, 25a) in Reihe geschaltet sind.

12. Generator nach Anspruch 11, **dadurch gekennzeichnet, dass** die Wandlermodule (25, 25a) miteinander baugleich ausgebildet sind.

13. Generator nach Anspruch 11 oder 12, **dadurch gekennzeichnet, dass** die Sekundärwicklungen (32, 32a) wenigstens einiger der Wandlermodule (25, 25a) gleichsinnig in Reihe schaltet sind.

14. Generator nach einem der Ansprüche 11 bis 13, **dadurch gekennzeichnet, dass** die Sekundärwicklungen (32a, 32b) wenigstens einiger der Wandlermodule (25a, 25b) gegensinnig in Reihe schaltet sind.

15. Generator nach einem der Ansprüche 11 bis 14, **dadurch gekennzeichnet, dass** der Spannungsdetektor (36) an lediglich eines der Wandlermodule (25, 25a, 25b, 25c) angeschlossen ist.

## Claims

1. An electrosurgical generator (10), in particular for HF surgical applications,
having a converter module (25) which comprises an externally controlled switch (26) connected to a control circuit (31) and a transformer (28), the primary winding (27) of which is connected in series with the switch (26) and the secondary winding (32) of which is connected to an electrode (17) and a counter electrode (14), wherein a patient to be treated can be placed between the electrode (17) and the counter electrode (14),
having a voltage detector (36) which is connected to the switch (26) and is configured to detect the voltage (Uₚ) occurring, and which comprises an indicator device (24) that is configured to generate a signal dependent on the detected voltage (Uₚ).

2. The generator according to claim 1, **characterized in that** the externally controlled switch (26) is connected at one end to a reference potential (M) and at another end to a connection point (29), which is connected to an end of the primary winding (27), wherein the voltage detector (36) is connected to the connection point (29).

3. The generator according to anyone of the preceding claims, **characterized in that** the voltage detector (36) is a peak voltage detector.

4. The generator according to anyone of the preceding claims, **characterized in that** the voltage detector (36) is a sample-and-hold-circuit, wherein the voltage measurement performed by the voltage detector (36) is synchronized with the switching of the externally controlled switch (26).

5. The generator according to anyone of the preceding claims, **characterized in that** the voltage detector (36) comprises a storage capacitor (C) which is connected to the switch (26) via a charging circuit (37).

6. The generator according to claim 5, **characterized in that** the charging circuit (37) is formed by at least one diode, preferably a plurality of diodes that are connected in series with one another.

7. The generator according to claim 5 or 6, **characterized in that** the storage capacitor (C) is assigned a discharge circuit (42) which has a controllable discharge current path (43) connected in parallel to the storage capacitor (C).

8. The generator according to claim 7, **characterized in that** the discharge circuit (42) is connected to the control circuit (31) so that its discharge current path (43) can be alternately switched back and forth between non-conducting and conducting states by the control circuit (31).

9. The generator according to claim 8, **characterized in that** the control circuit (31) is configured to alternately open and block the externally controlled switch (26), wherein it is further configured to also block the discharge current path (43) when the switch (26) is blocked.

10. The generator according to claim 9, **characterized in that** the control circuit (31) is configured to temporarily open the discharge current path (42) prior to the blocking of the switch (26).

11. The generator according to anyone of the preceding claims, **characterized in that**, in addition to the converter module (25), at least one further converter module (25a) is provided, wherein the secondary windings (32, 32a) of the converter modules (25, 25a) are connected in series.

12. The generator according to claim 11, **characterized in that** the converter modules (25, 25a) are of identical construction.

13. The generator according to claim 11 or 12, **characterized in that** the secondary windings (32, 32a) of at least some of the converter modules (25, 25a) are connected in series with equal orientation.

14. The generator according to anyone of the claims 11 to 13, **characterized in that** the secondary windings (32a, 32b) of at least some of the converter modules (25a, 25b) are connected in series with opposite orientations.

15. The generator according to anyone of the claims 11 to 14, **characterized in that** the voltage detector (36) is connected to only one of the converter modules (25, 25a, 25b, 25c).

## Revendications

1. Générateur électrochirurgical (10), en particulier pour des applications de chirurgie haute fréquence,
avec un module de conversion (25), qui présente un interrupteur (26) à commande externe raccordé à un circuit de commande (31) et un transformateur (28) dont l'enroulement primaire (27) est branché en série avec l'interrupteur (26) et dont l'enroulement secondaire (32) est raccordé à une électrode (17) et à une contre-électrode (14), dans lequel un patient (13) à soigner peut être placé entre l'électrode (17) et la contre-électrode (14),
avec un détecteur de tension (36), qui est raccordé à l'interrupteur (26) et est mis au point pour détecter la tension (Uₚ) apparaissant, et qui présente un dispositif indicateur (24) qui est mis au point pour générer un signal dépendant de la tension (Uₚ) détectée.

2. Générateur selon la revendication 1, **caractérisé en ce que** l'interrupteur (26) à commande externe est connecté par une extrémité à un potentiel de référence (M) et par une autre extrémité à un point de raccordement (29), qui est raccordé à une extrémité de l'enroulement primaire (27), dans lequel le détecteur de tension (36) est connecté au point de raccordement (29).

3. Générateur selon l'une quelconque des revendications précédentes, **caractérisé en ce que** le détecteur de tension (36) est un détecteur de pic de tension.

4. Générateur selon l'une quelconque des revendications précédentes, **caractérisé en ce que** le détecteur de tension (36) est un circuit échantillonneur-bloqueur [sample-and-hold], dans lequel la mesure de tension exécutée par le détecteur de tension (36) est synchronisée avec la commutation de l'interrupteur (26) à commande externe.

5. Générateur selon l'une quelconque des revendications précédentes, **caractérisé en ce que** le détecteur de tension (36) contient un condensateur de stockage (C) qui est raccordé à l'interrupteur (26) par l'intermédiaire d'un circuit de charge (37).

6. Générateur selon la revendication 5, **caractérisé en ce que** le circuit de charge (37) est formé par au moins une, de préférence plusieurs diodes branchées en série les unes avec les autres.

7. Générateur selon la revendication 5 ou 6, **caractérisé en ce qu'**est associé au condensateur de stockage (C) un circuit de décharge (42), qui présente un chemin de courant de décharge (43) pouvant être commandé, qui est branché en parallèle par rapport au condensateur de stockage (C).

8. Générateur selon la revendication 7, **caractérisé en ce que** le circuit de décharge (42) est raccordé au circuit de commande (31), de telle sorte que son chemin de courant de décharge (43) peut être commuté alternativement en va-et-vient entre l'état non conducteur et l'état conducteur par le circuit de commande (31).

9. Générateur selon la revendication 8, **caractérisé en ce que** le circuit de commande (31) est mis au point pour ouvrir et verrouiller alternativement l'interrupteur (26) à commande externe, dans lequel il est en outre mis au point pour verrouiller également le chemin de courant de décharge (43) lorsque l'interrupteur (26) est verrouillé.

10. Générateur selon la revendication 9, **caractérisé en ce que** le circuit de commande (31) est mis au point pour ouvrir temporairement le chemin de courant de décharge (42) avant le verrouillage de l'interrupteur (26).

11. Générateur selon l'une quelconque des revendications précédentes, **caractérisé en ce qu'**en plus du module de conversion (25), au moins un autre module de conversion (25a) est prévu, dans lequel les enroulements secondaires (32, 32a) des modules de conversion (25, 25a) sont branchés en série.

12. Générateur selon la revendication 11, **caractérisé en ce que** les modules de conversion (25, 25a) sont identiques les uns aux autres dans leur conception.

13. Générateur selon la revendication 11 ou 12, **caractérisé en ce que** les enroulements secondaires (32, 32a) d'au moins certains des modules de conversion (25, 25a) sont branchés en série dans le même sens.

14. Générateur selon l'une quelconque des revendications 11 à 13, **caractérisé en ce que** les enroulements secondaires (32a, 32b) d'au moins certains des modules de conversion (25a, 25b) sont branchés en série dans des sens opposés.

15. Générateur selon l'une quelconque des revendications 11 à 14, **caractérisé en ce que** le détecteur de tension (36) n'est connecté qu'à un seul des modules de conversion (25, 25a, 25b, 25c).
